(19) Europäisches Patentamt
European Patent Office
Office européen des brevets

(11) **EP 4 597 100 A1**

(12) **EUROPEAN PATENT APPLICATION**
published in accordance with Art. 153(4) EPC

(43) Date of publication:
**06.08.2025 Bulletin 2025/32**

(21) Application number: **23872859.6**

(22) Date of filing: **12.09.2023**

(51) International Patent Classification (IPC):
***G01N 33/487*** (2006.01)   ***G01N 30/72*** (2006.01)

(52) Cooperative Patent Classification (CPC):
**G01N 30/72; G01N 33/487**

(86) International application number:
**PCT/KR2023/013629**

(87) International publication number:
**WO 2024/071740 (04.04.2024 Gazette 2024/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.09.2022 KR 20220122568**

(71) Applicant: **Innobation Bio Co., Ltd.**
**Seoul 03929 (KR)**

(72) Inventors:
• **KIM, Seung Ku**
**Yongin-si, Gyeonggi-do 16865 (KR)**
• **KIM, Ki Tae**
**Seoul 06148 (KR)**

(74) Representative: **J A Kemp LLP**
**80 Turnmill Street**
**London EC1M 5QU (GB)**

(54) **ARTIFICIAL INTELLIGENCE-BASED INFORMATION PROVISION METHOD FOR DIAGNOSING LUNG CANCER BY USING BIOMARKER FOR LUNG CANCER DIAGNOSIS AND CLINICAL INFORMATION OF TEST SUBJECT**

(57)    The present invention relates to a method for providing information for artificial intelligence-based lung cancer diagnosis using a biomarker(s) for diagnosing lung cancer and clinical information of a test subject, and more specifically, to a metabolomic biomarker(s) composition for diagnosing lung cancer comprising kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC), and a method for providing artificial intelligence-based information for diagnosing lung cancer using the metabolomic biomarker(s) and clinical information of a test subject.

As a result of establishing an artificial intelligence-based algorithm model for diagnosing lung cancer using the biomarkers selected in the present invention and clinical information for diagnosing lung cancer, it was confirmed that the early screening ability of lung cancer using the algorithm developed in this invention was 90 ~ 92% sensitivity, 93 ~ 95% specificity, and 92 ~ 93% accuracy, which was very high compared to the existing lung cancer screening method, so this invention can effectively provide information on lung cancer diagnosis.

EP 4 597 100 A1

**Description**

[Technical field]

The present invention relates to a method for providing information for artificial intelligence-based lung cancer diagnosis using a biomarker(s) for diagnosing lung cancer and clinical information of a test subject, and more specifically, to a metabolomic biomarker(s) composition for diagnosing lung cancer comprising kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC), and a method for providing artificial intelligence-based information for diagnosing lung cancer using the metabolomic biomarker(s) and clinical information of a test subject.

[Background art]

Cancer is a disease in which cells multiply indefinitely and disrupt normal cell function, most commonly in liver, lung, stomach, breast, colon, and ovarian cancers, but can occur in virtually any tissue.

Initially, cancer diagnosis was based on external changes in biological tissues caused by the growth of cancer cells, but in recent years, cancer diagnosis has been attempted using the detection of trace biomolecules present in the tissues or cells of living organisms, such as blood, glycol-chains, and DNA. However, the most commonly used methods of cancer diagnosis are tissue samples obtained through biopsy or imaging.

Among them, biopsies are painful for the patient, expensive, and require a long time to diagnose. In addition, if the patient has actual cancer, there is a risk that the biopsy process may cause the cancer to metastasize, and if the biopsy cannot obtain a tissue sample, the disease cannot be diagnosed until the suspected tissue is surgically removed.

Lung cancer, in particular, is one of the most lethal cancers in the world, and current diagnosis of lung cancer relies heavily on imaging methods (X-ray, CT, MRI, etc.). However, more than half of lung cancer patients are already inoperable at the time of diagnosis, and even if surgery is deemed feasible, many of them cannot be completely resected. Therefore, early diagnosis and treatment of lung cancer is of utmost importance in order to increase the cure rate of lung cancer, but lung cancer has limited markers useful for diagnosis, making such diagnosis difficult. Therefore, it is necessary to find cancer-specific markers present in biological samples and develop methods to diagnose cancer with high accuracy and precision using these markers.

In recent years, methods using artificial intelligence have been studied for more accurate cancer diagnosis. Machine learning or deep learning is mainly used for analysis using artificial intelligence (AI), and various studies are being conducted to utilize AI in the biofield (e.g. Korean Publication Patent No. 10-2014-0002149, Korean Registered Patent No. 10-2268963).

[Disclosure]

[Technical Problem]

Accordingly, the inventors have made a diligent effort to screen for markers that can more accurately diagnose lung cancer. As a result, we have selected a set of biomarkers comprising kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC), and found that the expression levels of these biomarkers showed different patterns in patients and controls. In addition, the quantitative values of the seven biomarkers and clinical information of patients and controls were analyzed using an artificial intelligence-based algorithm, and the diagnostic ability of lung cancer was improved, and the invention was completed.

Accordingly, an object of the present invention is to provide a biomarker composition for diagnosing lung cancer.

Another object of the present invention is to provide a composition for diagnosing lung cancer comprising an agent (substance) that measures the expression level of the biomarker, and a diagnostic kit for lung cancer using the composition.

Another object of the present invention is to provide a method for providing artificial intelligence-based information for diagnosing lung cancer using the biomarkers and clinical information of a test subject.

[Technical solution]

To fulfill the above purposes, the present invention provides a biomarker composition for diagnosing lung cancer comprising kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC).

In a preferred embodiment of the present invention, the biomarker may be extracted from blood.

In another preferred embodiment of the present invention, the blood may be whole blood, plasma, or serum.

To fulfill other purposes, the present invention provides a composition for diagnosing lung cancer, comprising an agent for measuring a level in the blood of the biomarker composition for diagnosing lung cancer.

In a preferred embodiment of the present invention, the agent for measuring the level of the biomarker composition may be an agent for mass spectrometry.

The present invention also provides a kit for diagnosing lung cancer comprising an agent for measuring the level in the blood of the biomarker composition for diagnosing lung cancer.

To fulfill another purpose, the invention provides a method for providing artificial intelligence-based information for diagnosing lung cancer comprising: (a) the step of measuring expression levels of biomarkers for diagnosing lung cancer from the blood of a test subject, wherein the biomarkers comprise kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and

(b) the step of applying expression levels of the biomarkers and clinical information of a test subject to a machine learning algorithm model.

In one preferred embodiment of the present invention, the blood in step (a) may be whole blood, plasma, or serum.

In another preferred embodiment of the present invention, the concentration of the biomarker in step (a) above may be obtained by mass spectrometry of the whole blood, plasma, or serum sample by mass peak area, more specifically by liquid chromatography-mass spectrometry (LC-MS), wherein the mass spectrometer may be any one of triple TOF, triple quadrupole, and MALDI TOF capable of quantitative measurement.

In another preferred embodiment of the present invention, in the step of applying to the algorithmic model above (b), the biomarker level in the blood of the test subject and the clinical information of the test subject may be input to the algorithmic model to output as an output whether the test subject has lung cancer.

In another preferred embodiment of the present invention, the clinical information in step (b) above may be one or more of the following selected from the group consisting of age, BMI, and smoking history.

In another preferred embodiment of the present invention, the algorithmic model of step (b) above comprises or can be derived from:

(1) measuring levels of the biomarker for diagnosing lung cancer from blood of a group of lung cancer patient and a group of normal control, wherein the biomarker comprises kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and

(2) training the levels of the biomarker, and clinical information of lung cancer patient and control with a machine learning algorithm to generate a lung cancer incidence prediction model.

In another preferred embodiment of the present invention, the artificial intelligence may be machine learning or deep learning, and more specifically, the algorithm in step (b) above may be any one of a linear or nonlinear classification algorithm selected from the group consisting of a k-nearest neighbor algorithm; a logistic regression algorithm; a discriminant analysis algorithm; a partial least squares discriminant analysis algorithm; a support vector machine algorithm; a decision tree algorithm; a decision tree ensemble algorithm; and a neural network algorithm.

In another preferred embodiment of the present invention, where the algorithm is a support vector machine algorithm, the kernel function may be represented by the following Equation 1:

[Equation 1]

$$K\left(x_i, x_j\right) = \exp\left(-\gamma \|x_i - x_j\|^2\right)$$

$\chi$: Measured blood level of a biomarker composition for lung cancer diagnosis and clinical information of the test subject

$\gamma$: parameter for the flexibility (curvature) of the decision boundary

The present invention also provides an artificial intelligence-based lung cancer diagnosis prediction device comprising: a measurement part for measuring the level of a biomarker for diagnosing lung cancer in the blood of a test subject; and a cancer diagnosis part for inputting the biomarker level and clinical information of the test subject into a trained artificial intelligence algorithm to determine whether the test subject has developed lung cancer.

[Advantageous Effects]

In this invention, 7 biomarkers that can diagnose lung cancer more accurately were selected, and an artificial intelligence-based algorithm for diagnosing lung cancer was established the above biomarkers, and clinical information of lung cancer patients and a group of controls. The early screening ability of lung cancer using the algorithm developed in this invention was 90 ~ 92% sensitivity, 93 ~ 95% specificity, and 92 ~ 93% accuracy, which was very high compared to the existing lung cancer screening method, so this invention can effectively provide information on lung cancer diagnosis.

[Mode of the Invention]

The present invention will now be described in detail.

**Biomarker composition for diagnosing lung cancer**

In one aspect, the present invention relates to a biomarker composition for diagnosing lung cancer comprising kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC).

In the present invention, the biomarker can be derived from blood, wherein the blood may be whole blood, plasma, or serum.

As used in the present invention, the term "diagnosis" refers to the identification of the presence or characterization of a pathological condition. For the purposes of the present invention, diagnosis can be the identification of the presence or absence of lung cancer.

As used in the present invention, the term "diagnostic biomarker" refers to an organic biomolecule, such as a polypeptide, nucleic acid (e.g., mRNA, etc.), lipid, glycolipid, glycoprotein, sugar (monosaccharide, disaccharide, oligosaccharide, etc.), or the like, that shows a significant increase or significant decrease in a group of lung cancer patients compared to a group of normal controls, and is preferably a biomarker composition for diagnosing lung cancer.

In a specific embodiment of the present invention, blood from a control group (Con) and a lung cancer (LC) patient group was obtained, and the concentrations of KN, HC, OC, DC, DDC, MC, and PC in the blood were measured.

It was confirmed that the concentrations of the biomarkers of the present invention in the blood were significantly different from the quantitative values of the lung cancer patients and the normal controls, and it was confirmed that the concentrations of KN metabolite was increased and the concentrations of HC, OC, DC, DDC, MC, and PC metabolites were decreased in the blood of the lung cancer patients compared to the normal controls (Table 1 and Table 2).

**Composition for diagnosing lung cancer**

In another aspect, the present invention relates to a composition for diagnosing lung cancer comprising an agent for measuring levels in the blood of a biomarker composition for diagnosing lung cancer of the present invention.

Specific details of the composition for diagnosing lung cancer according to the present invention can refer to "Biomarker composition for diagnosing lung cancer" above.

The biomarkers of the present invention may be metabolites, and the agent for measuring the level(s) of the biomarker composition may be an agent for mass spectrometry.

The agent for mass spectrometry means an agent capable of analyzing the mass of a marker in whole blood, plasma, or serum, and more specifically, an agent capable of performing liquid chromatography-mass spectrometry (LC-MS).

**Kit for diagnosing lung cancer**

In another aspect, the present invention relates to a kit for diagnosing lung cancer comprising an agent for measuring a level in the blood of the biomarker composition for diagnosing lung cancer of the present invention.

Specific details of the composition for diagnosing lung cancer according to the present invention can refer to "Biomarker composition for diagnosing lung cancer" above.

The kits can be prepared by conventional methods known in the art. The kits may include, for example, antibodies in lyophilized form, buffers, stabilizers, inactive proteins, and the like.

**Method of providing information for diagnosing lung cancer using artificial intelligence-based algorithm**

In another consistent aspect, the present invention relates to a method for providing artificial intelligence-based information for diagnosing lung cancer comprising:

(a) the step of measuring levels of biomarkers for diagnosing lung cancer from the blood of a test subject, wherein the biomarkers comprise kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and

(b) the step of applying the expression levels of the biomarkers and clinical information of a test subject to a machine learning algorithm model.

In the present invention, the blood in step (a) may be whole blood, plasma, or serum.
In the present invention, the level (concentration) of the biomarker in step (a) above may be obtained by mass spectrometric analysis of the whole blood, plasma, or serum sample by mass peak area, more specifically by liquid chromatography-mass spectrometry (LC-MS), wherein the mass spectrometer may be any one of triple TOF, triple quadrupole, and MALDI TOF capable of quantitative measurement.
In the present invention, the step (b) of applying to the algorithmic model may include inputting the level of the biomarker in the blood of the test subject and the clinical information of the test subject into the algorithmic model to output whether the test subject has lung cancer as an output value.
In the present invention, the clinical information in step (b) may be one or more of the following selected from the group consisting of age, BMI, and smoking history.
In the present invention, the algorithmic model of step (b) above may comprise or can be derived from:

(1) measuring levels of the biomarker for diagnosing lung cancer from blood of a group of lung cancer patient and a group of normal control, wherein the biomarker comprises kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and

(2) training the levels of the biomarker, and clinical information of lung cancer patient and control with a machine learning algorithm to generate a lung cancer incidence prediction model.

In the present invention, the artificial intelligence may be machine learning or deep learning, and more specifically, the algorithm in step (b) above may be any one of linear or nonlinear classification algorithms selected from the group consisting of k-nearest neighbor algorithms; logistic regression algorithms; discriminant analysis algorithms; partial least squares discriminant analysis algorithms; support vector machine algorithms; decision tree algorithms; decision tree ensemble algorithms; and neural network algorithms.
In the present invention, where the algorithm is a support vector machine algorithm, the kernel function may be represented by the following Equation 1:

[Equation 1]

$$K(\boldsymbol{x}_i, \boldsymbol{x}_j) = \exp\left(-\gamma \|\boldsymbol{x}_i - \boldsymbol{x}_j\|^2\right)$$

$\chi$: Measured blood level of a biomarker composition for lung cancer diagnosis and clinical information of the test subject

$\gamma$: parameter for the flexibility (curvature) of the decision boundary

In a specific embodiment of the present invention, a prediction model was developed using quantitative values of seven biomarkers of the present invention measured in the blood of lung cancer patients and a group of controls and clinical information of lung cancer patients and a group of controls, and the algorithm was a support vector machine using a radial basis function as a kernel. The developed prediction model was tested for its ability to early diagnose lung cancer and found to have a sensitivity of 90~92%, specificity of 93~95%, and accuracy of 92~93%.
In other words, it was confirmed that the present AI-based lung cancer diagnosis method using the seven biomarkers and the clinical information of the test subjects has a very high accuracy compared to other existing diagnostic methods.

In another aspect, the present invention also provides an artificial intelligence-based lung cancer diagnosis prediction device comprising: a measurement part for measuring the level of a biomarker for diagnosing lung cancer in the blood of a test subject, wherein the biomarker comprises kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and

a cancer diagnosis part for inputting the biomarker level and clinical information of the test subject into a trained artificial intelligence algorithm to determine whether the test subject has developed lung cancer.

The present invention will now be described in more detail with reference to the following examples.

These embodiments are intended solely to illustrate the invention, and it will be apparent to one of ordinary skill in the art that the scope of the invention is not to be construed as limited by these embodiments.

**Example 1: Measurement of biomarker concentrations in the blood of lung cancer patients and controls**

1-1: Sample preparation

To determine whether the biomarker of the present invention can diagnose lung cancer, a total of 152 people were screened, specifically 92 normal controls and 60 lung cancer (LC) patients were screened and blood was collected through Ajou University Hospital. In addition, clinical information on age, BMI, and smoking history of LC patients and controls were collected.

1-2: Measuring biomarker concentrations in the blood

From the blood sample, plasma was separated and standard materials for each concentration required for the biomarker standard curves of kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC) were prepared.

20 $\mu$l of plasma and each standard material were vortexed with 400 $\mu$l of lipid extraction buffer (lipid extraction buffer; Abnova, Taiwan) and centrifuged (10,000g, 5 min, 4°C) after vortexing. After centrifugation, all supernatant were transferred to a new tube and dried using a concentrator for 12-16 hours. To the dried metabolome extract, 50 $\mu$l of 100% methanol with 0.1% formic acid (FA) was added, dissolved well using a vortexer, and analyzed using liquid chromatography-mass spectrometry/mass spectrometry (LC-MS/MS).

The LC used was a Shimadzu LC 40 system and the MS was an AB Sciex Triple Quad 5500+ system. The MS was equipped with a turbo spray ion source. The analytes were separated on a BEH C18 column (1.7 $\mu$m, 2.1*50 mm; Waters) of the Shimadzu LC 40 system. The solvent used was a two-step linear gradient (solvent A, 0.1% FA in water; solvent B, 0.1% FA in 100% acetonitrile; 5-55% solvent B 2.5 min, 55% solvent B 5.5 min, 55-95% solvent B 7.5 min, 95% solvent B 11 min, 95-5% solvent B 11.1 min, and 5% solvent B 14.5 min).

Mass spectrometry (MS/MS) was performed using multiple reaction monitoring (MRM) mode. The area of the mass peak with the same mass value in the mass spectrum at the same time period as the time when the metabolite corresponding to each biomarker passed through the liquid chromatography was calculated. A standard curve was constructed using the mass peaks of each biomarker standard and the mass peaks of each sample were plotted against the standard curve to determine the concentration of each biomarker.

[Table 1]

| Biomarker levels and clinical information for 92 controls | | | | | | | | | | | | |
|---|---|---|---|---|---|---|---|---|---|---|---|---|
| | KN (ng/ml) | HC (ng/ml) | OC (ng/ml ) | DC (ng/ml) | DDC (ng/ml ) | MC (ng/ml) | PC (ng/ml) | BMI (kg/m$^2$) | Age (year) | Curre ntly smoki ng | Past smok ing | Non-Smok ing |
| AJ_C0_ 001 | 565.97 | 11.03 | 119.36 | 178.99 | 36.36 | 11.2 | 20.79 | 26.30 | 56 | N | N | Y |
| AJ_C0_ 002 | 696.75 | 6.11 | 58.37 | 105.78 | 24.21 | 6.9 | 12.06 | 20.70 | 46 | N | N | Y |
| AJ_C0_ 003 | 438.06 | 11.78 | 87.14 | 140.65 | 37.46 | 17.74 | 32.98 | 35.93 | 43 | N | N | Y |
| AJ_C0_ 004 | 533.59 | 7.18 | 53.86 | 115.73 | 23.75 | 8.99 | 23.59 | 24.80 | 42 | Y | N | N |
| AJ_C0_ 005 | 436.29 | 7.33 | 72.2 | 142.21 | 24.93 | 8.96 | 23.15 | 24.88 | 55 | N | N | Y |
| AJ_C0_ 006 | 292.99 | 10.32 | 50.8 | 98.9 | 39.18 | 16.11 | 26.63 | 22.06 | 55 | N | N | Y |
| AJ_C0_ 007 | 426.71 | 8.54 | 43.89 | 75.56 | 11.71 | 4.29 | 13.13 | 25.62 | 44 | Y | N | N |
| AJ_C0_ 008 | 368.79 | 7.07 | 45.51 | 96.38 | 15.78 | 6.44 | 15.58 | 25.39 | 48 | N | N | Y |
| AJ_C0_ 009 | 487 | 11.88 | 144.67 | 188.41 | 28.47 | 9.62 | 19.89 | 26.45 | 54 | N | N | Y |
| AJ_C0_ 010 | 360.15 | 9.53 | 99.36 | 137.95 | 30.38 | 11.35 | 28.31 | 22.31 | 52 | N | N | Y |
| AJ_C0_ 011 | 422.1 | 6.9 | 33.36 | 42.7 | 9.49 | 3.38 | 10.05 | 31.25 | 59 | N | N | Y |
| AJ_C0_ 012 | 428.28 | 10.32 | 74.05 | 129.19 | 22.74 | 6.52 | 16.93 | 21.22 | 49 | N | N | Y |
| AJ_C0_ 013 | 407.99 | 9.21 | 58.55 | 118.85 | 21.02 | 10.56 | 34.34 | 24.68 | 49 | Y | N | N |
| AJ_C0_ 014 | 563.48 | 5.85 | 38.88 | 73.7 | 20.07 | 7.81 | 26.49 | 24.39 | 51 | Y | N | N |
| AJ_C0_ 015 | 586.51 | 24.49 | 207.65 | 373.13 | 60.73 | 15.7 | 26.16 | 25.56 | 58 | N | N | Y |
| AJ_C0_ 016 | 408.39 | 14.18 | 100.14 | 219.56 | 38.02 | 11.2 | 23.33 | 21.27 | 24 | N | N | Y |
| AJ_C0_ 017 | 513.77 | 7.41 | 109.3 | 162.96 | 33.47 | 8.59 | 17.42 | 22.34 | 46 | Y | N | N |
| AJ_C0_ 018 | 473.41 | 4.35 | 41.71 | 56.92 | 12.2 | 4.52 | 14.33 | 24.30 | 29 | N | Y | N |
| AJ_C0_ 021 | 500.4 | 7.55 | 52.12 | 107.11 | 30.15 | 10.5 | 26.4 | 22.04 | 48 | Y | N | N |
| AJ_C0_ 022 | 488.95 | 10.14 | 57.6 | 157.15 | 26.62 | 8.82 | 22.8 | 25.95 | 34 | Y | N | N |
| AJ_C0_ 023 | 493.44 | 7.3 | 57.09 | 111.53 | 22.49 | 8.4 | 20.71 | 23.31 | 46 | N | Y | N |
| AJ_C0_ 024 | 314.51 | 11.34 | 61.07 | 130.52 | 17.26 | 7.97 | 18.43 | 26.01 | 31 | Y | N | N |
| AJ_C0_ 025 | 350.2 | 4.29 | 29.11 | 44.98 | 9.49 | 4.44 | 14.45 | 23.23 | 28 | N | N | Y |
| AJ_C0_ 026 | 506.61 | 9.88 | 77.33 | 132.54 | 26.41 | 9.02 | 25.9 | 23.03 | 40 | Y | N | N |
| AJ_C0_ 027 | 406.21 | 11.29 | 65.99 | 122.33 | 18.67 | 7.56 | 20.47 | 23.73 | 48 | N | N | Y |

EP 4 597 100 A1

| Biomarker levels and clinical information for 92 controls | | | | | | | | | | | | |
|---|---|---|---|---|---|---|---|---|---|---|---|---|
| | KN (ng/ml) | HC (ng/ml) | OC (ng/ml ) | DC (ng/ml) | DDC (ng/ml ) | MC (ng/ml) | PC (ng/ml) | BMI (kg/m$^2$) | Age (year) | Curre ntly smoki ng | Past smok ing | Non-Smok ing |
| AJ_C0_ 028 | 395.3 | 20.78 | 115.77 | 242.94 | 39.02 | 16.82 | 25.5 | 21.51 | 60 | N | N | Y |
| AJ_C0_ 029 | 572.01 | 9.95 | 96.79 | 163.38 | 30.02 | 9.46 | 22.99 | 27.72 | 36 | Y | N | N |
| AJ_C0_ 030 | 467.9 | 7.84 | 63.68 | 114.65 | 24.59 | 7.66 | 18.52 | 22.99 | 51 | Y | N | N |
| AJ_C0_ 031 | 379.16 | 12.76 | 108.03 | 193.86 | 29.51 | 6.86 | 17.27 | 19.53 | 30 | N | Y | N |
| AJ_C0_ 032 | 572.43 | 9.37 | 62.48 | 107.88 | 16.09 | 4.95 | 18.67 | 24.91 | 27 | Y | N | N |
| AJ_C0_ 033 | 508.39 | 7.11 | 64.74 | 110.1 | 32.64 | 13.87 | 27.77 | 24.22 | 60 | N | N | Y |
| AJ_C0_ 034 | 352.66 | 5.28 | 28.58 | 48.5 | 9.66 | 6.59 | 20.59 | 25.06 | 56 | N | N | Y |
| AJ_C0_ 035 | 610.52 | 10.67 | 56.96 | 99.03 | 26.09 | 9.32 | 25.29 | 25.31 | 44 | N | N | Y |
| AJ_C0_ 036 | 386.25 | 11.13 | 63.71 | 119.96 | 21.55 | 7.65 | 19.53 | 22.77 | 48 | Y | N | N |
| AJ_C0_ 037 | 385.18 | 12.46 | 105.1 | 193.38 | 36.76 | 12.13 | 21.79 | 23.67 | 42 | N | N | Y |
| AJ_C0_ 038 | 588.1 | 7.82 | 57.37 | 120.29 | 39.32 | 9.75 | 17.01 | 22.39 | 52 | Y | N | N |
| AJ_C0_ 039 | 281.52 | 5.75 | 29.2 | 47.55 | 8.02 | 2.93 | 10.9 | 26.17 | 54 | N | N | Y |
| AJ_C0_ 040 | 617.28 | 8.8 | 44.77 | 71.32 | 21.06 | 10.04 | 29.71 | 24.80 | 47 | N | Y | N |
| AJ_C0_ 041 | 496.65 | 4.36 | 28.06 | 45.47 | 8.43 | 3.33 | 14.33 | 22.66 | 44 | N | N | Y |
| AJ_C0_ 042 | 460.22 | 10.79 | 76.7 | 125.79 | 21.63 | 6.36 | 14.02 | 22.77 | 49 | N | N | Y |
| AJ_C0_ 043 | 630.54 | 9.51 | 63.47 | 129.91 | 33.03 | 9.04 | 32.76 | 32.05 | 31 | N | N | Y |
| AJ_C0_ 044 | 580.12 | 3.92 | 15.2 | 29.32 | 9.9 | 5 | 16.27 | 21.09 | 30 | N | N | Y |
| AJ_C0_ 045 | 407.92 | 11.87 | 132.96 | 242.53 | 39.76 | 11.51 | 19.9 | 20.03 | 33 | N | N | Y |
| AJ_C0_ 046 | 401.11 | 7.37 | 60.65 | 94.22 | 14.17 | 3.71 | 15.08 | 28.34 | 33 | Y | N | N |
| AJ_C0_ 047 | 469.21 | 5.47 | 38.96 | 60.97 | 12.58 | 4.51 | 13.26 | 23.71 | 55 | N | N | Y |
| AJ_C0_ 048 | 323.31 | 9.87 | 54.81 | 90.38 | 19.12 | 6.2 | 18.23 | 23.39 | 30 | N | N | Y |
| AJ_C0_ 050 | 301.96 | 10.82 | 74.89 | 123.6 | 21.22 | 7.56 | 22.77 | 30.67 | 39 | Y | N | N |
| AJ_C0_ 051 | 324.31 | 6.42 | 53.2 | 145.12 | 27.52 | 10.18 | 22.66 | 23.30 | 34 | Y | N | N |
| AJ_C0_ 052 | 384.26 | 7.27 | 41.33 | 92.8 | 17.22 | 6.59 | 16.63 | 22.34 | 44 | N | Y | N |
| AJ_C0_ 053 | 502.84 | 8.83 | 57.94 | 110.16 | 26.53 | 9.91 | 17.82 | 22.43 | 55 | N | N | Y |

(continued)

| Biomarker levels and clinical information for 92 controls | | | | | | | | | | | | |
|---|---|---|---|---|---|---|---|---|---|---|---|---|
| | KN (ng/ml) | HC (ng/ml) | OC (ng/ml ) | DC (ng/ml) | DDC (ng/ml ) | MC (ng/ml) | PC (ng/ml) | BMI (kg/m$^2$) | Age (year) | Curre ntly smoki ng | Past smok ing | Non- Smok ing |
| AJ_C0_ 054 | 411.24 | 4.03 | 28.25 | 76.55 | 19.63 | 7.62 | 18.84 | 22.99 | 38 | N | N | Y |
| AJ_C0_ 055 | 615.82 | 10.92 | 91.37 | 188.04 | 83.01 | 23.15 | 26.69 | 26.89 | 43 | N | N | Y |
| AJ_C0_ 056 | 600.29 | 9.35 | 60.86 | 125.79 | 21.6 | 8.55 | 18.52 | 25.35 | 52 | Y | N | N |
| AJ_C0_ 058 | 500.84 | 5.15 | 51.43 | 103.72 | 15.42 | 6.42 | 13 | 18.37 | 32 | N | N | Y |
| AJ_C0_ 059 | 461.25 | 10.41 | 100.38 | 172.28 | 43.59 | 13.7 | 27.26 | 21.14 | 50 | N | N | Y |
| AJ_C0_ 060 | 452.22 | 8.15 | 38.51 | 80.73 | 14.2 | 7.31 | 18.89 | 23.32 | 46 | N | Y | N |
| AJ_C0_ 062 | 631.25 | 8.04 | 66.25 | 125.73 | 31.26 | 10.82 | 19.98 | 20.96 | 48 | N | N | Y |
| AJ_C0_ 063 | 324.83 | 6.26 | 62.25 | 109.83 | 18.13 | 6.2 | 13.85 | 22.16 | 20 | N | N | Y |
| AJ_C0_ 064 | 648.19 | 9.24 | 60.19 | 137.48 | 35.46 | 11.8 | 24.33 | 24.93 | 74 | N | N | Y |
| AJ_C0_ 065 | 431.81 | 11.34 | 116.62 | 256.97 | 48.25 | 15.78 | 21.41 | 23.12 | 30 | Y | N | N |
| AJ_C0_ 066 | 318.39 | 6.98 | 108.53 | 176.19 | 19.86 | 5.08 | 11.87 | 23.50 | 34 | N | N | Y |
| AJ_C0_ 068 | 458.44 | 8.48 | 41.4 | 91.29 | 19.45 | 8.43 | 20.11 | 24.90 | 47 | N | N | Y |
| AJ_C0_ 069 | 311.41 | 0.91 | 4.34 | 7.75 | 1.82 | 2.78 | 9.15 | 23.87 | 55 | N | N | Y |
| AJ_C0_ 070 | 544.56 | 3.64 | 16.22 | 32.72 | 7.38 | 4.01 | 10.16 | 21.23 | 56 | N | N | Y |
| AJ_C0_ 071 | 584.79 | 11.18 | 106 | 210.36 | 42.93 | 9.62 | 21.74 | 21.97 | 47 | N | Y | N |
| AJ_C0_ 072 | 969.7 | 2.03 | 9.77 | 18.79 | 7.29 | 3.38 | 8.64 | 21.78 | 43 | N | N | Y |
| AJ_C0_ 073 | 496.04 | 6.42 | 49.48 | 103.9 | 18.03 | 6.03 | 12.43 | 23.94 | 44 | Y | N | N |
| AJ_C0_ 075 | 637.52 | 3.05 | 18.85 | 39.54 | 8.1 | 3.3 | 11.95 | 22.49 | 39 | N | Y | N |
| AJ_C0_ 076 | 477.39 | 9.96 | 112.09 | 186.51 | 29.9 | 11.87 | 18.97 | 21.37 | 31 | N | N | Y |
| AJ_C0_ 077 | 483.01 | 8.84 | 57.9 | 102.29 | 24.52 | 9.05 | 26.2 | 24.61 | 47 | N | N | Y |
| AJ_C0_ 078 | 477.42 | 11.03 | 110.4 | 188.46 | 30.69 | 8.09 | 16.7 | 24.16 | 52 | N | Y | N |
| AJ_C0_ 079 | 588.62 | 2.14 | 16.29 | 28.91 | 7.01 | 2.67 | 9.36 | 20.76 | 51 | N | N | Y |
| AJ_C0_ 080 | 305.91 | 9.8 | 94.06 | 188.71 | 40.97 | 9.83 | 17.28 | 21.97 | 60 | N | Y | N |
| AJ_C0_ 081 | 269.2 | 14.01 | 125.65 | 193.07 | 25.24 | 8.93 | 24 | 18.49 | 32 | N | N | Y |
| AJ_C0_ 082 | 402.95 | 7.27 | 45.58 | 93.65 | 16.08 | 7.15 | 16.36 | 23.12 | 36 | N | Y | N |

| Biomarker levels and clinical information for 92 controls | | | | | | | | | | | |
|---|---|---|---|---|---|---|---|---|---|---|---|
| | KN (ng/ml) | HC (ng/ml) | OC (ng/ml ) | DC (ng/ml) | DDC (ng/ml ) | MC (ng/ml) | PC (ng/ml) | BMI (kg/m$^2$) | Age (year) | Curre ntly smoki ng | Past smok ing | Non-Smok ing |
| AJ_C0_ 083 | 387.6 | 4.42 | 33.38 | 56.55 | 11.27 | 6.03 | 16.37 | 23.67 | 34 | N | N | Y |
| AJ_C0_ 084 | 469.66 | 7.09 | 57.6 | 141.56 | 32.95 | 10.77 | 18.92 | 21.51 | 52 | N | N | Y |
| AJ_C0_ 086 | 403.1 | 6.4 | 74.33 | 127.04 | 18.69 | 6.07 | 13.05 | 19.61 | 49 | N | N | Y |
| AJ_C0_ 087 | 585.66 | 4.45 | 29.75 | 56.11 | 11.9 | 5.82 | 22.82 | 25.06 | 40 | N | N | Y |
| AJ_C0_ 088 | 597.5 | 8.47 | 69.87 | 115 | 20.63 | 5.96 | 13.52 | 23.18 | 53 | N | Y | N |
| AJ_C0_ 089 | 487.13 | 8.94 | 89.04 | 166.68 | 40.3 | 8.43 | 17.59 | 20.45 | 35 | N | N | Y |
| AJ_C0_ 090 | 456.43 | 7.38 | 70.17 | 131.01 | 25.49 | 6.29 | 16.12 | 27.76 | 36 | Y | N | N |
| AJ_C0_ 091 | 344.56 | 3.01 | 21.39 | 40.43 | 9.26 | 5.02 | 16.94 | 22.47 | 40 | Y | N | N |
| AJ_C0_ 092 | 378.63 | 16.21 | 87.11 | 215.1 | 41 | 16.65 | 32.61 | 21.71 | 32 | Y | N | N |
| AJ_C0_ 093 | 518.51 | 19.64 | 77.95 | 135.31 | 36.99 | 21.55 | 22.63 | 24.98 | 56 | N | N | Y |
| AJ_C0_ 094 | 461.52 | 5.74 | 30.3 | 32.96 | 6.6 | 5.73 | 19.06 | 25.80 | 34 | N | N | Y |
| AJ_C0_ 095 | 395.75 | 6.06 | 49.08 | 81.19 | 12.75 | 6.8 | 21.7 | 23.53 | 60 | N | N | Y |
| AJ_C0_ 096 | 499.94 | 10.84 | 75.9 | 137.11 | 35.41 | 9.98 | 21.23 | 24.51 | 52 | N | N | Y |
| AJ_C0_ 097 | 361.78 | 9.47 | 90.25 | 148.82 | 24.48 | 7.26 | 20.65 | 22.32 | 47 | N | N | Y |
| AJ_C0_ 098 | 578.23 | 9.78 | 75.95 | 122.27 | 19.04 | 6.91 | 21.02 | 26.84 | 34 | N | N | Y |
| AJ_C0_ 099 | 256.85 | 12.01 | 115.23 | 190.66 | 34.46 | 9.66 | 17.75 | 21.45 | 28 | N | N | Y |
| AJ_C0_ 100 | 597.88 | 10.22 | 99.83 | 181.02 | 32.58 | 6.49 | 15.51 | 21.37 | 44 | N | N | Y |

[Table 2]

Biomarker levels and clinical information for 60 patients with lung cancer

| | KN (ng/ml) | HC (ng/ml) | OC (ng/ml) | DC (ng/ml) | DDC (ng/ml) | MC (ng/ml) | PC (ng/ml) | BMI (kg/m²) | Age (year) | Currently smoking | Past smoking | Non-Smoking |
|---|---|---|---|---|---|---|---|---|---|---|---|---|
| AJ_C3_051 | 450.06 | 4.82 | 26.24 | 37.13 | 11.61 | 10.03 | 25.11 | 28.27 | 73 | N | Y | N |
| AJ_C3_052 | 401.32 | 2.34 | 10.57 | 16.86 | 4.65 | 4.86 | 12.83 | 24.31 | 72 | N | N | Y |
| AJ_C3_053 | 347.12 | 4.18 | 22.9 | 34.65 | 9.04 | 8.41 | 21.06 | 24.68 | 44 | Y | N | N |
| AJ_C3_054 | 186.9 | 3.88 | 12.06 | 22.16 | 11.83 | 10.97 | 17.92 | 20.13 | 44 | N | N | Y |
| AJ_C3_055 | 360.27 | 4.99 | 27.15 | 34.99 | 13.69 | 8.83 | 16.33 | 21.35 | 79 | N | N | Y |
| AJ_C3_056 | 591.25 | 4.67 | 25.85 | 41.71 | 13.76 | 9.66 | 24.06 | 21.61 | 75 | Y | N | N |
| AJ_C3_057 | 378.64 | 2.79 | 19.17 | 26.71 | 7.09 | 5.89 | 16.05 | 31.62 | 63 | N | N | Y |
| AJ_C3_058 | 281.45 | 2.53 | 13.21 | 19.55 | 6.15 | 6.95 | 15.08 | 24.96 | 60 | N | N | Y |
| AJ_C3_059 | 386.16 | 3.95 | 20.81 | 28.67 | 6.18 | 6.02 | 13.7 | 24.24 | 65 | N | N | Y |
| AJ_C3_060 | 226.18 | 4.64 | 31.58 | 40.15 | 11.49 | 9.21 | 20.63 | 24.09 | 75 | N | N | Y |
| AJ_C3_061 | 346.31 | 4.91 | 24.94 | 31.66 | 9.93 | 8.24 | 16.45 | 22.03 | 71 | N | N | Y |
| AJ_C3_062 | 287.85 | 3.26 | 18.62 | 25.9 | 7.75 | 6.14 | 13.74 | 30.86 | 53 | N | N | Y |
| AJ_C3_063 | 267.3 | 6.25 | 22.91 | 36.18 | 12.32 | 11.86 | 28.98 | 29.24 | 66 | N | N | Y |
| AJ_C3_064 | 313.23 | 3.76 | 22.39 | 28.07 | 9.93 | 7.21 | 19.98 | 21.08 | 56 | N | N | Y |
| AJ_C3_065 | 313.44 | 2.7 | 14.46 | 16.47 | 5.14 | 4.95 | 13.51 | 22.93 | 52 | N | N | Y |
| AJ_C3_066 | 592.1 | 6.17 | 36.69 | 61.93 | 21.1 | 15.43 | 19.98 | 18.00 | 64 | N | Y | N |
| AJ_C3_067 | 281.51 | 3.35 | 17.73 | 24.01 | 7.58 | 5.42 | 11.17 | 27.34 | 61 | Y | N | N |
| AJ_C3_068 | 472.92 | 7.66 | 44.26 | 70.61 | 18.97 | 10.5 | 18.97 | 29.45 | 60 | Y | N | N |
| AJ_C3_069 | 447.73 | 8.7 | 51.91 | 85.22 | 19.55 | 13.8 | 29.8 | 18.81 | 71 | Y | N | N |
| AJ_C3_070 | 839.73 | 6.78 | 48.98 | 66.58 | 12.79 | 6.57 | 20.91 | 22.64 | 74 | N | N | Y |
| AJ_C3_071 | 624.36 | 11.32 | 90.88 | 157.2 | 29.66 | 16.45 | 25.02 | 26.22 | 77 | N | Y | N |
| AJ_C3_072 | 355.86 | 5.77 | 31.61 | 44.2 | 12.91 | 11.41 | 24.12 | 28.96 | 55 | N | N | Y |
| AJ_C3_073 | 261.79 | 4.51 | 15.07 | 23.73 | 7.9 | 8.34 | 20.36 | 25.73 | 71 | N | Y | N |
| AJ_C3_074 | 441.76 | 3.85 | 15.53 | 24.95 | 6.42 | 6.85 | 15.29 | 28.61 | 64 | N | N | Y |
| AJ_C3_075 | 449.71 | 4.45 | 34.81 | 51.87 | 15.9 | 11.6 | 21.15 | 22.07 | 79 | N | N | Y |

(continued)

Biomarker levels and clinical information for 60 patients with lung cancer

| | KN (ng/ml) | HC (ng/ml) | OC (ng/ml) | DC (ng/ml) | DDC (ng/ml) | MC (ng/ml) | PC (ng/ml) | BMI (kg/m$^2$) | Age (year) | Currently smoking | Past smoking | Non-Smoking |
|---|---|---|---|---|---|---|---|---|---|---|---|---|
| AJ_C3_076 | 245.65 | 5.55 | 48.38 | 66.66 | 14.18 | 8.46 | 16.82 | 23.87 | 57 | N | N | Y |
| AJ_C3_077 | 469.79 | 4.82 | 33.43 | 59.81 | 22.14 | 13.18 | 32.33 | 26.71 | 76 | Y | N | N |
| AJ_C3_078 | 309.96 | 1.82 | 10.33 | 15.28 | 6.4 | 4.58 | 14.67 | 26.35 | 67 | N | N | Y |
| AJ_C3_079 | 351.11 | 5.73 | 35.04 | 70.47 | 26.41 | 15.01 | 15.58 | 20.83 | 54 | N | N | Y |
| AJ_C3_080 | 670.59 | 5.34 | 28.58 | 40.13 | 7.88 | 7.45 | 20.42 | 23.05 | 50 | Y | N | N |
| AJ_C3_081 | 468.59 | 4.13 | 23.23 | 39.73 | 11.46 | 9.89 | 25.79 | 23.38 | 71 | N | N | Y |
| AJ_C3_082 | 311.03 | 3.86 | 19.15 | 30.03 | 10.98 | 10.29 | 20.16 | 22.10 | 74 | Y | N | N |
| AJ_C3_083 | 436.59 | 4.29 | 17.28 | 28.84 | 15.62 | 12.66 | 21.07 | 20.05 | 76 | Y | N | N |
| AJ_C3_084 | 315.24 | 4.87 | 28.97 | 41.49 | 10.78 | 6.19 | 17.27 | 27.00 | 46 | Y | N | N |
| AJ_C3_085 | 354.14 | 2.81 | 14.31 | 23.59 | 8.88 | 8.04 | 15.04 | 23.71 | 67 | Y | N | N |
| AJ_C3_086 | 421.44 | 7.02 | 27.1 | 44 | 13.67 | 10.81 | 16.99 | 23.38 | 71 | N | Y | N |
| AJ_C3_087 | 315.73 | 3.06 | 15.55 | 25.58 | 7.59 | 5.4 | 15.78 | 23.84 | 47 | Y | N | N |
| AJ_C3_088 | 230.25 | 7.3 | 13.7 | 23.44 | 16.02 | 14.42 | 17.74 | 19.07 | 65 | N | Y | N |
| AJ_C3_089 | 272.29 | 2.67 | 17.57 | 22.02 | 4.29 | 3.07 | 8.27 | 19.63 | 77 | N | N | Y |
| AJ_C3_090 | 348.47 | 2.29 | 7.98 | 16.02 | 6.33 | 4.5 | 11.91 | 21.66 | 70 | N | N | Y |
| AJ_C3_091 | 320.17 | 2.37 | 14.15 | 21.81 | 18.77 | 12.85 | 24.36 | 22.46 | 52 | Y | N | N |
| AJ_C3_092 | 374.72 | 2.8 | 15.79 | 23.31 | 12.08 | 8.95 | 19.65 | 23.23 | 69 | N | Y | N |
| AJ_C3_093 | 352.25 | 4.09 | 27.77 | 48.86 | 11.34 | 6.9 | 13.31 | 23.73 | 62 | N | N | Y |
| AJ_C3_094 | 371.51 | 6.76 | 27.69 | 47.77 | 17 | 12.29 | 20.96 | 25.67 | 72 | Y | N | N |
| AJ_C3_095 | 310.36 | 4.16 | 21.99 | 39.12 | 15.9 | 14.84 | 19.86 | 24.57 | 68 | N | N | Y |
| AJ_C3_096 | 444.68 | 5.64 | 34.43 | 48.83 | 18.45 | 14.41 | 22.12 | 21.23 | 70 | Y | N | N |
| AJ_C3_097 | 493.41 | 6.18 | 19.1 | 30.61 | 10.12 | 8.33 | 14.54 | 20.58 | 75 | Y | N | N |
| AJ_C3_098 | 397.11 | 5.4 | 32.8 | 46.32 | 16.93 | 10.33 | 21.73 | 25.15 | 58 | Y | N | N |
| AJ_C3_099 | 579.11 | 3.91 | 26.1 | 36.22 | 9.28 | 7.23 | 19.38 | 25.26 | 63 | Y | N | N |
| AJ_C3_100 | 300.36 | 2.85 | 16.97 | 21.73 | 5 | 4.51 | 12.09 | 22.89 | 51 | Y | N | N |

(continued)

| Biomarker levels and clinical information for 60 patients with lung cancer | | | | | | | | | | | | |
|---|---|---|---|---|---|---|---|---|---|---|---|---|
| | KN (ng/ml) | HC (ng/ml) | OC (ng/ml) | DC (ng/ml) | DDC (ng/ml ) | MC (ng/ml) | PC (ng/ml ) | BMI (kg/m$^2$) | Age (year) | Curre ntly smok ing | Past smok ing | Non- Smok ing |
| AJ_C3_ 101 | 379.52 | 3.76 | 18.88 | 32.42 | 14.67 | 10.97 | 18.18 | 26.20 | 57 | Y | N | N |
| AJ_C3_ 102 | 363.74 | 6.05 | 46.31 | 63.64 | 16.32 | 9.44 | 20.62 | 30.62 | 77 | N | N | Y |
| AJ_C3_ 103 | 397 | 5.62 | 23.34 | 36.22 | 15.35 | 10.99 | 19.16 | 17.65 | 60 | N | N | Y |
| AJ_C3_ 104 | 188.24 | 4.36 | 22.95 | 30.56 | 8.59 | 5.27 | 17.12 | 23.07 | 80 | N | N | Y |
| AJ_C3_ 105 | 221.58 | 1.73 | 7.63 | 10.07 | 3.18 | 3.17 | 9.02 | 15.43 | 70 | N | N | Y |
| AJ C3_ 106 | 227.28 | 5.63 | 31.03 | 44.21 | 15.1 | 8.17 | 24.99 | 26.09 | 64 | Y | N | N |
| AJ_C3_ 107 | 691.67 | 23.29 | 120.87 | 177.79 | 43.26 | 14.99 | 14.22 | 23.45 | 72 | N | Y | N |
| AJ_C3_ 108 | 462.73 | 3.96 | 19.02 | 31.38 | 8.2 | 6.85 | 18.35 | 26.04 | 66 | Y | N | N |
| AJ_C3_ 109 | 150.57 | 1.76 | 3.9 | 8.58 | 3.82 | 5.53 | 13.69 | 22.81 | 51 | N | N | Y |
| AJ_C3_ 110 | 287.81 | 5.05 | 20.59 | 31.13 | 12.63 | 9.08 | 14.38 | 20.34 | 55 | N | N | Y |

As shown in Table 1 and Table 2, the blood concentrations of the seven biomarkers were significantly different between the quantitative values of the lung cancer patients and the control group, and the concentration of KN metabolite was increased and the concentrations of HC, OC, DC, DDC, MC, and PC metabolites were decreased in the blood of the lung cancer patients compared to the control group.

**Example 2: Developing an AI-powered algorithmic model for lung cancer diagnosis**

In this invention, a support vector machine algorithm using a radial basis function as a kernel was applied to the quantitative values of 7 biomarkers, and clinical information (age and BMI) of lung cancer patients and controls to develop a prediction model to diagnose whether lung cancer has occurred.
We trained a lung cancer incidence prediction model using the kernel function represented by Equation 1 below and tuning the algorithm parameters:

[Equation 1]

$$K(\boldsymbol{x}_i, \boldsymbol{x}_j) = \exp\left(-\gamma \|\boldsymbol{x}_i - \boldsymbol{x}_j\|^2\right)$$

$\chi$: Measured blood level of a biomarker composition for lung cancer diagnosis and clinical information of the test subject

$\gamma$: parameter for the flexibility (curvature) of the decision boundary

The parameter gamma (γ) in Equation 1 determines the range of influence of a single training sample, while C, another parameter of the support vector machine that is independent of the kernel function, determines how much training samples are allowed to be misclassified. Since both parameters underfit or overfit the learning model depending on their values, the optimal parameters were selected through iterative cross-validation.
The results of the discrimination of lung cancer using the support vector machine model trained with quantitative values of seven biomarkers measured for 629 lung cancer patient samples and 511 control samples, and clinical information (age and smoking history) collected together are shown in Table 3.

[Table 3]

| Examples of sensitivity, specificity, and accuracy of LC screening decision based on C and gamma (γ) values of the support vector machine algorithm | | | | |
|---|---|---|---|---|
| C | γ | Sensitivity | Specificity | Accuracy |
| 46.12556 | 0.015910 | 91.73% | 94.91% | 93.16% |
| 42.57584 | 0.024607 | 91.10% | 95.50% | 93.07% |
| 39.49145 | 0.022897 | 91.10% | 95.11% | 92.89% |
| 74.34696 | 0.017166 | 91.41% | 95.11% | 93.07% |
| 54.75801 | 0.001124 | 90.46% | 93.15% | 91.67% |

As a result of checking the ability to early diagnose lung cancer using the developed prediction model, it was found that the sensitivity was 90 ~ 92%, specificity was 93 ~ 95%, and accuracy was 92 ~ 93%, as shown in Table 3 above. In other words, it was confirmed that the AI-based lung cancer diagnosis method using the seven biomarkers and clinical information of the present invention has a very high accuracy compared to other existing diagnostic methods.
In this invention, it was confirmed that the early screening ability of lung cancer using the algorithm developed in this invention was 90 ~ 92% sensitivity, 93 ~ 95% specificity, and 92 ~ 93% accuracy, which was very high compared to the existing lung cancer screening method, so this invention can effectively provide information on lung cancer diagnosis.

**Claims**

**1.** A biomarker composition for diagnosing lung cancer comprising kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC).

2. The biomarker composition for diagnosing lung cancer of claim 1, wherein the biomarker is derived from blood.

3. The biomarker composition for diagnosing lung cancer of claim 1, wherein the blood is whole blood, plasma, or serum.

4. A composition for diagnosing lung cancer, comprising an agent for measuring a level in the blood of the biomarker composition for diagnosing lung cancer of any one of claims 1 to 3.

5. The composition for diagnosing lung cancer of claim 4, wherein the agent for measuring the level of the biomarker composition is an agent for mass spectrometry.

6. A kit for diagnosing lung cancer, comprising an agent for measuring a level in the blood of a biomarker composition for diagnosing lung cancer of any one of claims 1 to 3.

7. A method of providing information for diagnosing lung cancer using artificial intelligence, comprising:

   (a) the step of measuring levels of biomarkers for diagnosing lung cancer from the blood of the test subject, wherein the biomarkers comprise kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and
   (b) the step of applying the levels of the biomarkers and clinical information of a test subject to a machine learning algorithm model.

8. The method of providing information for diagnosing lung cancer using artificial intelligence of claim 7, wherein the blood in step (a) is whole blood, plasma, or serum.

9. The method of providing information for diagnosing lung cancer using artificial intelligence of claim 7, wherein the measuring levels of biomarkers in step (a) is obtained via liquid chromatography-mass spectrometry (LC-MS).

10. The method of providing information for diagnosing lung cancer using artificial intelligence of claim 7, wherein the applying to the algorithmic model in step (b) is to input levels of the biomarkers in the blood of a test subject and clinical information into the algorithmic model and output as an output whether the test subject has lung cancer.

11. The method of providing information for diagnosing lung cancer using artificial intelligence of claim 7, wherein the clinical information in step (b) is at least one selected from the group consisting of age, BMI, and smoking history.

12. The method of providing information for diagnosing lung cancer using artificial intelligence of claim 7, wherein the algorithmic model in step (b) comprises:

    (i) the step of measuring levels of biomarkers for diagnosing lung cancer from the blood of the test subject, wherein the biomarkers comprise kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and
    (2) training the levels of the biomarkers and clinical information of a group of lung cancer patients and a group of controls with a machine learning algorithm to generate a lung cancer incidence prediction model.

13. The method of providing information for diagnosing lung cancer using artificial intelligence of claim 7, wherein the algorithm of step (b) is any one of a linear or nonlinear classification algorithm selected from the group consisting of a k-nearest neighbor algorithm; a logistic regression algorithm; a discriminant analysis; a partial least squares discriminant analysis; a support vector machine algorithm; a decision tree algorithm; a decision tree ensemble algorithm; and a neural network algorithm.

14. The method of providing information for diagnosing lung cancer using artificial intelligence of claim 7, where the algorithm of step (b) is a support vector machine algorithm, the kernel function is represented by the following Equation 1:

[Equation 1]

$$K\left(\boldsymbol{x}_i, \boldsymbol{x}_j\right) = \exp\left(-\gamma\|\boldsymbol{x}_i - \boldsymbol{x}_j\|^2\right)$$

in Equation 1, $\chi$ is a measured blood level of the biomarker composition for lung cancer diagnosis and clinical information of the test subject, and $\gamma$ is a parameter for the flexibility (curvature) of the decision boundary.

**15.** An artificial intelligence-based lung cancer diagnosis prediction device comprising:

(i) a measurement part for measuring the level of a biomarker for diagnosing lung cancer in the blood of a test subject, wherein the biomarker comprises kynurenine (KN), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and
(ii) a cancer diagnosis part for inputting the biomarker level and clinical information of the test subject into a trained artificial intelligence algorithm to determine whether the test subject has developed lung cancer.

## INTERNATIONAL SEARCH REPORT

| International application No. |
|---|
| **PCT/KR2023/013629** |

### A. CLASSIFICATION OF SUBJECT MATTER

**G01N 33/487**(2006.01)i; **G01N 30/72**(2006.01)i

According to International Patent Classification (IPC) or to both national classification and IPC

### B. FIELDS SEARCHED

Minimum documentation searched (classification system followed by classification symbols)

G01N 33/487(2006.01); G01N 30/00(2006.01); G01N 30/72(2006.01); G01N 30/86(2006.01); G01N 33/48(2006.01); G06N 3/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 키뉴레닌(kynurenine), 헥사노일-L-카르니틴(hexanoyl-L-carnitine), 옥타노일-L-카르니틴(octanoyl-L-carnitine), 데카노일-L-카르니틴(decanoyl-L-carnitine), 도데카노일-L-카르니틴(dodecanoyl-L-carnitine), 미리스토일-L-카르니틴(myristoyl-L-carnitine), 팔미토일-L-카르니틴(palmitoyl-L-carnitine), 바이오마커(biomarker), 폐암(lung cancer), 진단(diagnosis), 인공지능(artificial intelligence)

### C. DOCUMENTS CONSIDERED TO BE RELEVANT

| Category* | Citation of document, with indication, where appropriate, of the relevant passages | Relevant to claim No. |
|---|---|---|
| Y | XIE, Ying et al. Early lung cancer diagnostic biomarker discovery by machine learning methods. Translational Oncology. 17 November 2020 (online publication date), vol. 14, no. 1, article no. 100907, pp. 1-10.<br>    See abstract; pages 2-5; and figures 2A and 4. | 1-15 |
| Y | KR 10-2020-0116410 A (NATIONAL CANCER CENTER) 12 October 2020 (2020-10-12)<br>    See paragraphs [0028]-[0029] and [0031]. | 1-15 |
| Y | NI, Junjun et al. Simultaneous determination of thirteen kinds of amino acid and eight kinds of acylcarnitine in human serum by LC-MS/MS and its application to measure the serum concentration of lung cancer patients. Biomedical Chromatography. 08 June 2016 (online publication date), vol. 30, no. 11, pp. 1796-1806 (inner pp. 1-11).<br>    See page 2; and table 6. | 1-15 |

☑ Further documents are listed in the continuation of Box C.    ☑ See patent family annex.

| | | | |
|---|---|---|---|
| \* | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **09 January 2024** | **10 January 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2023/013629** |

### C.    DOCUMENTS CONSIDERED TO BE RELEVANT

| Category* | Citation of document, with indication, where appropriate, of the relevant passages | Relevant to claim No. |
| --- | --- | --- |
| Y | MORENO, Paula et al. Metabolomic profiling of human lung tumor tissues-nucleotide metabolism as a candidate for therapeutic interventions and biomarkers. Molecular Oncology. 13 September 2018 (online publication date), vol. 12, pp. 1778-1796.<br>    See abstract; page 1779; and figure 4. | 1-15 |
| A | WO 2022-073502 A1 (SUN YAT-SEN UNIVERSITY CANCER CENTER (THE AFFILIATED CANCER HOSPITAL OF SUN YAT-SEN UNIVERSITY, THE CANCER RESEARCH INSTITUTE OF SUN YAT-SEN UNIVERSITY)) 14 April 2022 (2022-04-14)<br>    See entire document. | 1-15 |
| PX | KR 10-2569168 B1 (INNOBATION BIO CO., LTD.) 24 August 2023 (2023-08-24)<br>    See claims 1 and 3-14. | 1-15 |

Form PCT/ISA/210 (second sheet) (July 2022)

## INTERNATIONAL SEARCH REPORT
### Information on patent family members

International application No.

**PCT/KR2023/013629**

| Patent document cited in search report | | | Publication date (day/month/year) | Patent family member(s) | | | Publication date (day/month/year) |
|---|---|---|---|---|---|---|---|
| KR | 10-2020-0116410 | A | 12 October 2020 | CN | 113939737 | A | 14 January 2022 |
| | | | | EP | 4027139 | A2 | 13 July 2022 |
| | | | | JP | 2022-531621 | A | 07 July 2022 |
| | | | | KR | 10-2022-0062238 | A | 16 May 2022 |
| | | | | KR | 10-2395558 | B1 | 10 May 2022 |
| | | | | US | 2022-0349895 | A1 | 03 November 2022 |
| | | | | WO | 2020-204373 | A2 | 08 October 2020 |
| WO | 2022-073502 | A1 | 14 April 2022 | CN | 112979664 | A | 18 June 2021 |
| | | | | CN | 113960235 | A | 21 January 2022 |
| | | | | CN | 114334828 | A | 12 April 2022 |
| | | | | CN | 114338403 | A | 12 April 2022 |
| | | | | CN | 116381073 | A | 04 July 2023 |
| | | | | EP | 4015520 | A1 | 22 June 2022 |
| | | | | EP | 4207645 | A1 | 05 July 2023 |
| | | | | JP | 2023-504178 | A | 01 February 2023 |
| | | | | KR | 10-2022-0136349 | A | 07 October 2022 |
| | | | | US | 11225594 | B1 | 18 January 2022 |
| | | | | US | 2022-0115385 | A1 | 14 April 2022 |
| | | | | US | 2022-0315598 | A1 | 06 October 2022 |
| | | | | US | 2023-0194554 | A1 | 22 June 2023 |
| | | | | US | 2023-0261940 | A1 | 17 August 2023 |
| | | | | WO | 2021-109737 | A1 | 10 June 2021 |
| | | | | WO | 2022-073368 | A1 | 14 April 2022 |
| | | | | WO | 2022-073406 | A1 | 14 April 2022 |
| | | | | WO | 2022-073453 | A1 | 14 April 2022 |
| KR | 10-2569168 | B1 | 24 August 2023 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020140002149
- KR 102268963